# FASCICULE DE BREVET EUROPEEN

(11) **EP 2 094 383 B1**
(45) Date de publication et mention de la délivrance du brevet: **12.05.2010**
(21) Numéro de dépôt: 07858666.6
(22) Date de dépôt: 06.11.2007
(51) Int. Cl.: B01J 19/02, C07B 39/00

(54) **REACTEURS REVETUS, LEUR PROCEDE DE FABRICATION ET LEURS UTILISATIONS**
BESCHICHTETE REAKTOREN, ZUGEHÖRIGES HERSTELLUNGSVERFAHREN UND IHRE VERWENDUNG
COATED REACTORS, PRODUCTION METHOD THEREOF AND USE OF SAME

(30) Priorité: 14.11.2006 FR 0654878
(43) Date de publication de la demande: 02.09.2009
(73) Titulaire: ARKEMA FRANCE, 92700 Colombes (FR)
(72) Inventeur: DEVIC, Michel, 69110 Sainte Foy Les Lyon (FR)
(74) Mandataire: Dang, Doris
(86) Numéro de dépôt international: PCT/FR2007/052304
(87) Numéro de publication internationale: WO 2008/059154

(56) Documents cités:
- FR-A- 1 376 529
- FR-A- 2 277 004
- FR-A1- 2 374 433
- US-A1- 2004 101 448

## Description

### Domaine de l'invention

La présente invention se rapporte à des réacteurs revêtus résistant à la corrosion acide, leur procédé de fabrication et leurs utilisations dans des procédés en milieu superacide.

### Art antérieur et problème technique

Les réactions en milieu superacide, en particulier les réactions de fluoration en phase liquide nécessitent pour être efficaces d'utiliser un milieu réactionnel riche en HF et en SbCl₅ (ou SbClₓF_{y}) et des températures élevées (80 à 120°C). L'HF anhydre sous forme de phase liquide forme avec SbCl₅ un milieu superacide très corrosif. Les métaux et alliages usuels anti-corrosion comme les aciers inoxydables, les inconels, le nickel, les hastelloy, etc. n'ont pas une résistance suffisante pour réaliser un réacteur industriel.

Une solution (JP 07-233102) consiste à appliquer un revêtement en polymère fluoré à l'intérieur du réacteur en acier inox. Une autre solution (US 4166536, US 3824115) consiste à utiliser un polymère fluoré contenant des particules de substances inorganiques telles que de la silice, du graphite ou du carbone.

Cependant, l'application de ce type de revêtement à l'intérieur du réacteur soulève de nombreux problèmes techniques comme le souligne le brevet WO 99/00344:
- Les dépôts de polymères obtenus par pulvérisation et fusion de poudre de polymère sont poreux, le métal est attaqué par l'HF et le revêtement se décolle.
- Les dépôts obtenus par fusion et rotomoulage sont plus épais et étanches, mais cette technique se limite aux réacteurs de petites dimensions (3785 litres) et, de plus, ces revêtements même épais sont encore légèrement perméables et des acides finissent par pénétrer entre la couche de polymère et la paroi en métal du réacteur et des surpressions se créent et provoquent des gonflements et des déformations importantes du revêtement en polymère fluoré.

Le brevet WO 99/00344 propose d'évacuer ces surpressions par le percement de petits trous dans la paroi du réacteur (0,31 cm à 1,27 cm de diamètre).

L'utilisation d'un revêtement en polymère fluoré dans un réacteur industriel n'est en outre possible à ce jour qu'à faible température (20 à 40°C) car le coefficient de dilatation des polymères fluorés est très supérieur à celui de l'acier. Aux températures nécessaires à la fluoration en phase liquide des chloroalcanes (80 à 120°C), la dilatation du revêtement est très importante et provoque des désordres structurels (plis, tension, déformation, déchirures, arrachements) aggravés par la faible résistance mécanique du polymère à chaud.

Par ailleurs, les problèmes de dilatation différentielle entre le polymère et le métal dans les réacteurs qui entraînent des décollements et arrachements du revêtement sont connus. Des solutions qui utilisent des revêtements multicouches de polymères fluorés, et de résine (US 3779854) et de fibres de verre existent mais sont totalement inadaptées à la mise en oeuvre de réactions en milieu super acide tel que l'HF.

Jusqu'à maintenant donc, aucune solution satisfaisante n'a été trouvée pour réaliser des réacteurs résistant sur le plan chimique et mécanique à des milieux corrosifs super acides.

L'invention a pour but de proposer des réacteurs revêtus résistant à la fois sur le plan mécanique et chimique aux milieux corrosifs acides.

L'invention se rapporte donc à un réacteur comprenant une paroi interne métallique sur laquelle est ancré un revêtement en polymère fluoré, l'ancrage étant assuré par une tôle perforée metallique située entre la paroi interne métallique et le revêtement en polymère fluoré, et la face de ladite tôle en contact avec la paroi métallique du réacteur présente une rugosité suffisante pour servir d'espace libre (pour les gaz) entre celle-ci et la paroi métallique du réacteur ; le réacteur est muni d'un dispositif pour permettre le maintien de la pression dans l'espace libre inférieure à celle du réacteur.

Des orifices peuvent être percés dans la paroi métallique du réacteur pour contrôler ladite pression.

Les bords des trous de la tôle en contact avec le revêtement en polymère fluoré sont de préférence légèrement arrondis afin d'éviter tout cisaillement qui pourrait abîmer le revêtement.

La tôle perforée peut être munie de nervures verticales, lesquelles sont de préférence disposées régulièrement.

Les nervures de section, de préférence semi-circulaire ou trapézoïdale, et avantageusement de 0,1 à 1 cm² sont ménagées, notamment par emboutissage ou pliage lors de la fabrication de la tôle perforée, dans des zones de la tôle ne comportant pas de trou. L'espacement entre les nervures est de préférence compris entre 10 et 50 cm.

L'épaisseur du revêtement en polymère fluoré peut être de 1 à 10 mm et de préférence 1,5 à 5 mm.

Les polymères fluorés (PF) utilisés dans l'invention sont des polymères thermoplastiques résistant aux milieux acides notamment choisis dans le groupe consistant en le polychloro-trifluoroéthylène (PCTFE), les copolymères du tétrafluoroéthylène et du perfluoropropène (FEP), les copolymères du tétrafluoroéthylène et du perfluoro-propylvinyl-ether (PFA), les copolymères du tétrafluoroéthylène et de l'éthylène (ETFE), les polymères du trifluorochloroéthylène et de l'éthylène (E-CTFE) et leurs mélanges.

De préférence, le polymère fluoré utilisé est le copolymère de tétrafluoroéthylène et de hexafluoropropylène (FEP) pour ses propriétés de non diffusion de l'antimoine (Sb) dans le polymère. Le FEP utilisé présente de 10 à 15 % et de préférence 12 % en poids de hexafluoropropylène.

L'épaisseur de la tôle perforée peut être de 1 à 10 mm et de préférence 3 à 6 mm. Avantageusement, cette épaisseur est voisine de celle du revêtement en polymère fluoré.

Le diamètre des trous de la tôle perforée, lorsqu'ils sont circulaires, peut être de 10 à 50 mm et de préférence 15 à 30 mm.

Les trous peuvent aussi être de forme oblongue, carrée ou rectangulaire.

Les trous peuvent être réalisés par perçage et chanfrainage du bord, par poinçonnage ou par emboutissage.

La surface occupée par les trous peut représenter entre 10 et 50 % et de préférence entre 30 et 40 % de la surface totale de la tôle perforée.

La tôle métallique perforée est de préférence réalisée dans de l'acier inox.

Le procédé de fabrication du réacteur revêtu comprend une étape au cours de laquelle la paroi interne métallique du réacteur est mise en contact avec la face rugueuse d'une tôle perforée, et l'autre face libre de la tôle étant mise en contact avec le revêtement en polymère fluoré, suivie d'une étape d'ancrage au cours de laquelle le revêtement en polymère fluoré s'enfonce à travers les trous de la tôle et vient ainsi s'appuyer sur la paroi interne du réacteur sous l'action de la chaleur et de la pression.

La paroi interne du réacteur peut être revêtue sur sa totalité ou sur seulement la partie en contact avec le milieu corrosif (phase liquide). Avantageusement, la paroi interne n'est revêtue que sur la cuve du réacteur.

Le revêtement est assujetti de façon étanche sur le haut de la cuve au moyen de dispositifs habituels, par exemple: le bord supérieur du revêtement peut être mis en forme de collet battu, dont l'angle est de préférence compris entre 45° et 90°, entre un ou deux joints en polytetrafluoroéthylène (PTFE) comprimés par la mise en place du couvercle du réacteur.

La paroi intérieure du couvercle peut aussi comprendre un revêtement en FEP ou tout autre polymère fluoré résistant au milieu réactionnel superacide. Le revêtement peut être simplement fixé par des moyens conventionnels ou par ancrage comme décrit pour la cuve du réacteur.

Une ou plusieurs rainures circulaires, de préférence de section comprise entre 0,2 et 2 cm², peuvent être usinée(s) sur la paroi intérieure du réacteur, de préférence perpendiculairement aux nervures de la tôle perforée, afin de collecter les gaz recueillis par les nervures. Des orifices percés à travers la paroi métallique du réacteur permettent de relier ces rainures par des tuyaux au dispositif de contrôle de la pression régnant entre le revêtement et la paroi interne métallique du réacteur. Avantageusement un orifice est percé dans le fond du réacteur pour recueillir les condensats liquides.

Avantageusement une rainure est usinée au niveau de la bride du joint de cuve.

Les réacteurs revêtus tel que décrit ci-dessus sont capables de supporter des conditions de réactions en milieu super acide, en particulier les réactions de fluoration en phase liquide, telles que des températures allant de 0 à 150°C et de préférence 60 à 120°C et une pression de 1 à 15 bar absolus.

Pour améliorer la conductivité thermique d'un tel réacteur, le revêtement en polymère fluoré peut être chargé en nanotubes de carbone.

On désigne par nanotubes des tubes ou des fibres creuses de diamètre de l'ordre de 5 à 20 nanomètres (nm) et de longueur de l'ordre de 100 à 1000 fois le diamètre.

Le carbone possède trois formes allotropiques bien connues: le carbone amorphe, le graphite et le diamant. Le graphite se retrouve dans les fibres de carbone, très légères et résistantes. Le diamant est couramment utilisé pour ses propriétés mécaniques exceptionnelles, et pour sa forte conductivité thermique. Les nanotubes de carbone, nouvelle forme allotropique du carbone, sont considérés comme une espèce unique de systèmes carbonés situés à mi-chemin entre les fibres de carbone classiques et les nouvelles formes du carbone telles que les fullerènes. Leur rapport longueur sur diamètre est si grand qu'ils peuvent être considérés, à l'égard de certaines propriétés, comme des structures unidimensionnelles. Il existe deux types de nanotubes de carbone: les monofeuillets et les multifeuillets.

Diamètre : quelques nanomètres pour les monofeuillets et de l'ordre de 10 à quelques dizaines de nanomètres pour les multifeuillets.

Longueur : plusieurs micromètres.

Un nanotube de carbone monofeuillet, dans le cas où il est parfait, peut être défini comme un feuillet de graphène enroulé et refermé sur lui-même formant ainsi un cylindre constitué uniquement d'atomes de carbone. Les extrémités sont formées de deux demi-sphères carbonées.

Un nanotube multifeuillet est un empilement concentrique de nanotubes monofeuillets.

La présente invention a également pour objet un réacteur comprenant le revêtement en polymère fluoré chargé en nanotubes de carbone.

### Mode de réalisation

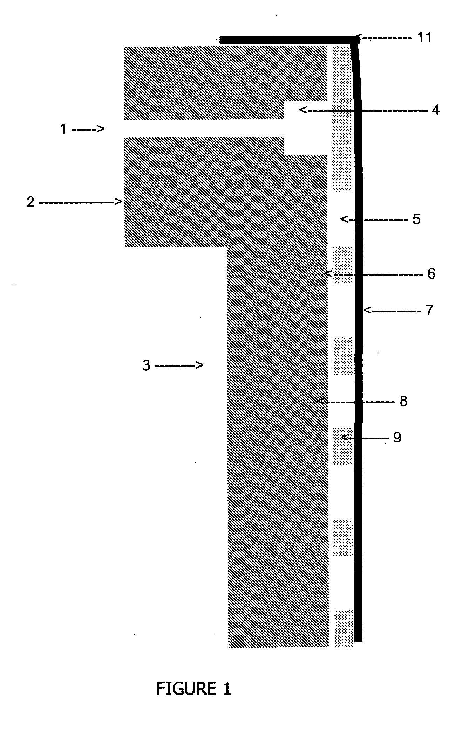

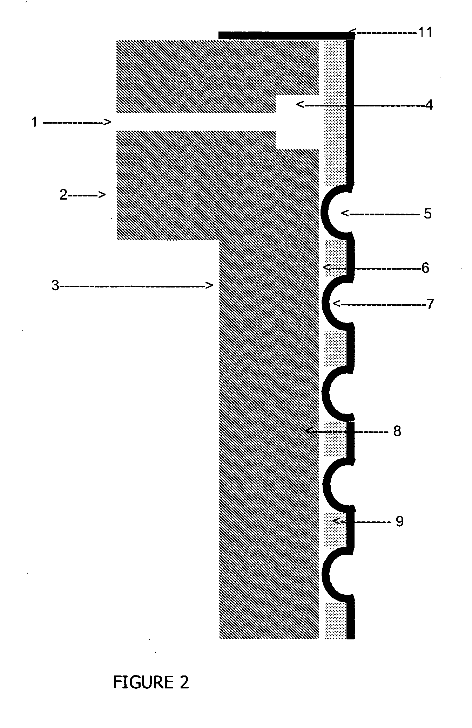

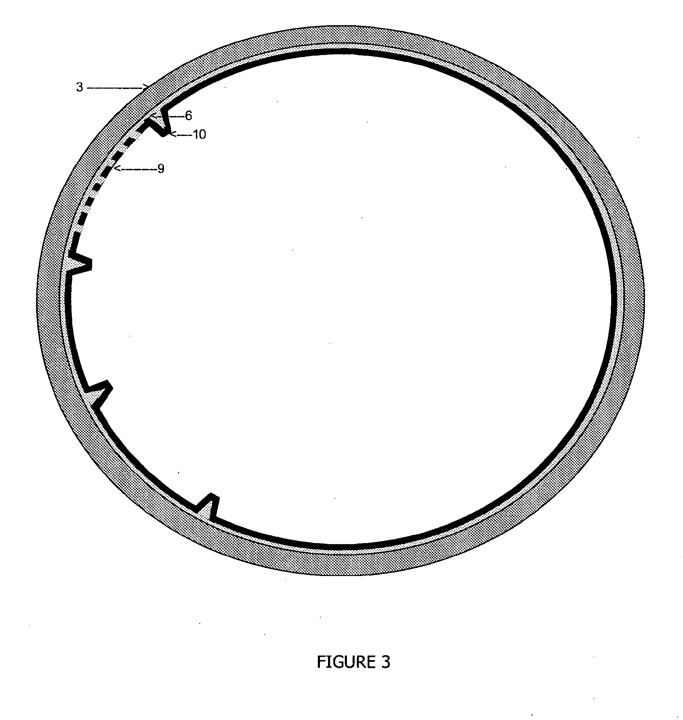

Un mode de réalisation particulier de l'invention est illustré à l'aide des figures 1 à 3.

La figure 1 est une coupe verticale du réacteur revêtu.

La figure 2 est une coupe verticale du réacteur revêtu après mise en service.

La figure 3 est une coupe horizontale de la cuve du réacteur avec la tôle perforée munie de nervures.

La face de la tôle perforée (9) comprenant des nervures (10) est ajustée et fixée, par plusieurs points de soudure, sur la paroi interne métallique (8) de la cuve (3) d'un réacteur, munie d'orifices reliés entre eux par des canalisations.

Un revêtement (7) constitué de plaques de FEP, soudées entre elles, est mis en place contre la face libre de tôle perforée ainsi fixée.

Une rainure (4) est usinée au niveau de la bride du joint de cuve (2) et permet de collecter les gaz provenant de l'espace libre (6) entre la paroi interne (8) et la tôle perforée (9) comportant des trous (5) et de relier cet espace libre au dispositif de contrôle de pression via l'orifice (1).

Le bord supérieur du revêtement est mis en forme de collet battu à 90° (11).

On contrôle ensuite, à l'aide d'une pompe à vide ou par l'introduction d'un gaz inerte, via l'orifice (1), la pression régnant dans l'espace libre créé entre la paroi interne métallique du réacteur de manière à assurer que cette pression est maintenue à une valeur inférieure à celle régnant à l'intérieur du réacteur.

Le réacteur est mis en service en le mettant sous pression (1 à 10 bar absolu), puis porté grâce à la double enveloppe chauffante à une température comprise entre 130 et 160°C. Ce chauffage permet de ramollir le revêtement et ainsi d'assurer une incrustation du revêtement dans les trous de la tôle perforée. Enfin, le réacteur est refroidi mais maintenu sous la même pression.

### Exemples

Matériels d'essais pour tester le revêtement :
- Une plaque de FEP de 2,3 mm d'épaisseur et de dimension 21 cm sur 30 cm.
- Une plaque en inox 316L de 5 mm d'épaisseur de dimension 25 cm sur 36 cm comprenant une double enveloppe avec circulation d'huile chaude sur sa face inférieure (simulation de la paroi intérieure du réacteur) et un orifice central relié à une pompe a vide.
- Un cadre métallique de dimension intérieure 19 cm sur 28 cm (extérieure 25x36 cm) pouvant être vissé en plusieurs points sur la face supérieure de la plaque d'inox.
- Une plaque de tôle perforée en acier, commercialisée par la société GANTOIS sous la référence R 25 T 33 et ayant les caractéristiques suivantes :
   - Dimensions : 21 cm sur 30 cm
   - Epaisseur : 3 mm
   - Diamètre des trous : 2,5 cm
   - Nombre de trous (à l'intérieur du cadre) : 48.

Le pourtour de la plaque de FEP posée sur la plaque en inox est fixée sur ladite plaque en inox d'une manière rigide et étanche par vissage du cadre métallique par-dessus la plaque de FEP.

La face inférieure de la plaque d'inox est munie d'une double enveloppe avec circulation d'huile chaude permettant ainsi de la chauffer. Un orifice au centre de la plaque d'inox relié à un tuyau permet de faire le vide entre la plaque de FEP et la plaque d'inox.

### Essai comparatif :

On chauffe la plaque de FEP mise en place comme ci-dessus sous vide et jusqu'à 160°C et on constate une déformation sous l'effet de la dilatation. Les bords de la plaque de FEP étant bloqués, des plis apparaissent au niveau du cadre. De plus, ces plis subsistent après refroidissement sous pression.

### Essai conforme à l'invention :

La plaque de tôle perforée en acier est prise en sandwich entre la plaque de FEP et la plaque d'inox massive et l'ensemble est serré dans le cadre métallique.

On réalise l'essai comme précédemment (c'est-à-dire : mise sous vide et chauffage de la plaque d'inox à 160°C).

On constate que sous l'action de la dépression (dans l'espace créé entre FEP et plaque inox) et du ramollissement du FEP à chaud, la plaque de FEP s'enfonce dans les trous de la tôle jusqu'à toucher la plaque d'inox. Par ailleurs, aucune autre déformation n'est observée et aucun pli n'apparaît au bord du cadre.

Après refroidissement sous vide, la plaque de FEP reste bien plane et légèrement incrustée dans chaque trou.

Au bout de 3 cycles successifs de chauffage à 160°C puis refroidissement sous vide, on ne constate toujours aucun désordre.

La déformation du FEP sous l'action de la dilatation a donc bien été contenue au voisinage des trous et ne s'est pas propagée sur toute la surface de la plaque en entraînant comme à l'essai précédent la formation de plis.

L'ancrage de la plaque de FEP est ainsi bien effectif et permet d'assurer le bon fonctionnement du revêtement à chaud.

### Matériel pour la mise en oeuvre au laboratoire de la réaction de fluoration

Un réacteur d'un litre comprenant une cuve en acier inox 316 L, de diamètre intérieur de 100 mm et de hauteur 153 mm, dans laquelle une rainure circulaire de 5 mm de largeur et 2 mm de profondeur est usinée en haut de la cuve (au niveau de la bride de fixation du couvercle). Un trou de 2 mm de diamètre est percé dans la bride relie la rainure à l'extérieur de la cuve.

Un cylindre en tôle d'inox perforée (épaisseur 2 mm, diamètre des trous : 3 mm, 4 trous par cm²) de 100 mm de diamètre extérieur, de 110 mm de haut. Ce cylindre est ajusté à l'intérieur de la cuve.

Un revêtement en FEP, constitué d'un cylindre obtenu par soudure d'une plaque en FEP de 1,5 mm d'épaisseur, d' un fond bombé en FEP de 1,5 mm soudé à l'extrémité inférieure du cylindre et d'un collet battu à 45° obtenu par thermoformage de l'extrémité supérieure du cylindre. Ce revêtement est ajusté à l'intérieur du cylindre en tôle perforée.

La pression derrière le revêtement est maintenue pendant tous les essais à la pression atmosphérique, on ne constate aucune fuite à la sortie de l'orifice percé dans la paroi du réacteur.

### Réaction de fluoration en batch du dichlorométhane :

Le réacteur ainsi constitué est chargé avec 120 g de SbCl₅, 160 g d'HF anhydre et de 170 g de CH₂Cl₂, on chauffe à 90°C pendant 5 h. De l'HCl se dégage et la pression est régulée à 9 bars.

Le TTG (conversion du dichlorométhane) est de 83 %, la sélectivité en F31 (chlorofluorométhane) est de 9,4 % et de 90,5 % en F32 (difluorométhane).

### Réaction de fluoration en batch du perchloroéthylène (PER) :

Le réacteur ainsi constitué est chargé avec 150 g de SbCl₅, 300 g d'HF et 83 g de PER. On chauffe à 100°C pendant 6 h sous une pression de 13 bars avec dégagement d'HCl.

Le TTG (conversion en PER) est de 99,9 % et la sélectivité en F123 (dichlorotrifluoroéthane) est de 96,3 %.

Ces essais sont effectués 18 fois pour le CH₂Cl₂ et 8 fois pour le PER. Après démontage du réacteur et de la tôle perforée on ne constate aucune corrosion de la paroi intérieure du réacteur.

Ces essais montrent que le revêtement en FEP est étanche au milieu réactionnel tres corrosif dans les conditions de réaction de fluoration en phase liquide sous pression et à chaud.

Le réacteur ainsi constitué permet de mettre en oeuvre de façon efficace les réactions de fluorations.

## Revendications

1. Réacteur revêtu comprenant une paroi interne métallique sur laquelle est ancré un revêtement en polymère fluoré, l'ancrage étant assuré par une tôle perforée située entre la paroi interne métallique et le revêtement en polymère fluoré, et la face de ladite tôle en contact avec la paroi métallique du réacteur présente une rugosité suffisante pour servir d'espace libre (pour les gaz) entre celle-ci et la paroi métallique du réacteur ; le réacteur est muni d'un dispositif pour permettre le maintien de la pression dans l'espace libre inférieure à celle du réacteur.

2. Réacteur selon la revendication 1 **caractérisé en ce que** le polymère fluoré est un copolymère de tétrafluoroéthylène et de hexafluoropropylène.

3. Réacteur selon la revendication 1 ou 2 **caractérisé en ce que** l'épaisseur du revêtement en polymère fluoré est de 1 à 10 mm et de préférence de 1,5 à 5 mm.

4. Réacteur selon l'une quelconque des revendications précédentes **caractérisé en ce que** l'épaisseur de la tôle perforée est de 1 à 10 mm et de préférence 3 à 6 mm.

5. Réacteur selon l'une quelconque des revendications précédentes **caractérisé en ce que** la surface occupée par les trous représente entre 10 et 50 % et de préférence entre 30 et 40 % de la surface totale de la tôle perforée.

6. Réacteur selon l'une quelconque des revendications précédentes **caractérisé en ce que** des orifices sont percés dans la paroi interne métallique du réacteur.

7. Réacteur selon l'une quelconque des revendications précédentes **caractérisé en ce que** la tôle perforée est munie de nervures verticales.

8. Réacteur selon l'une quelconque des revendications précédentes **caractérisé en ce qu'**une ou plusieurs rainures circulaires sont usinées sur la paroi intérieure du réacteur.

9. Réacteur selon l'une quelconque des revendications précédentes **caractérisé en ce que** le revêtement est chargé en nanotubes de carbone.

10. Procédé de fabrication d'un réacteur selon l'une quelconque des revendications précédentes **caractérisé en ce qu'**il comprend une étape au cours de laquelle la paroi interne métallique du réacteur est mise en contact avec la face rugueuse d'une tôle perforée, et l'autre face libre de la tôle étant mise en contact avec le revêtement en polymère fluoré, suivie d'une étape d'ancrage au cours de laquelle le revêtement en polymère fluoré s'enfonce à travers les trous de la tôle et vient ainsi s'appuyer sur la paroi interne du réacteur sous l'action de la chaleur et de la pression.

11. Procéde de fluoration en phase liquide **caractérisé en ce que** l'on met en oeuvre un réacteur selon l'une quelconque des revendications 1 à 9.

## Claims

1. Coated reactor comprising an inner metal wall anchored to which is a fluoropolymer coating, the anchoring being provided by a perforated sheet located between the inner metal wall and the fluoropolymer coating, and the face of said sheet in contact with the metal wall of the reactor has a sufficient roughness to act as a free space (for gases) between it and the metal wall of the reactor; the reactor is equipped with a device that makes it possible to maintain the pressure in the free space below that of the reactor.

2. Reactor according to Claim 1, **characterized in that** the fluoropolymer is a copolymer of tetrafluoroethylene and of hexafluoropropylene.

3. Reactor according to Claim 1 or 2, **characterized in that** the thickness of the fluoropolymer coating is from 1 to 10 mm and preferably from 1.5 to 5 mm.

4. Reactor according to any one of the preceding claims, **characterized in that** the thickness of the perforated sheet is from 1 to 10 mm and preferably 3 to 6 mm.

5. Reactor according to any one of the preceding claims, **characterized in that** the surface occupied by the holes represents between 10 and 50% and preferably between 30 and 40% of the total surface of the perforated sheet.

6. Reactor according to any one of the preceding claims, **characterized in that** orifices are made in the inner metal wall of the reactor.

7. Reactor according to any one of the preceding claims, **characterized in that** the perforated sheet is provided with vertical ribs.

8. Reactor according to any one of the preceding claims, **characterized in that** one or more circular grooves are machined in the inner wall of the reactor.

9. Reactor according to any one of the preceding claims, **characterized in that** the coating is filled with carbon nanotubes.

10. Method for producing a reactor according to any one of the preceding claims, **characterized in that** it comprises a step during which the inner metal wall of the reactor is brought into contact with the rough face of a perforated sheet, and the other free face of the sheet being brought into contact with the fluoropolymer coating, followed by an anchoring step during which the fluoropolymer coating sinks through the holes of the sheet and thus comes to rest on the inner wall of the reactor under the action of heat and pressure.

11. Liquid-phase fluorination method, **characterized in that** a reactor according to any one of Claims 1 to 9 is used.

## Patentansprüche

1. Beschichteter Reaktor, der eine metallische Innenwand umfasst, auf der eine Beschichtung aus fluoriertem Polymer verankert ist, wobei die Verankerung durch ein gelochtes Blech gewährleistet wird, das zwischen der metallischen Innenwand und der Beschichtung aus fluoriertem Polymer angeordnet ist, wobei die Seite des Blechs im Kontakt mit der metallischen Wand des Reaktors eine ausreichende Unebenheit aufweist, um als freier Zwischenraum (für die Gase) zwischen dem Blech und der metallischen Wand des Reaktors zu dienen; und wobei der Reaktor mit einer Vorrichtung ausgestattet ist, die es ermöglicht, in dem freien Zwischenraum einen Druck beizubehalten, der unter dem Druck des Reaktors liegt.

2. Reaktor nach Anspruch 1, **dadurch gekennzeichnet, dass** das fluorierte Polymer ein Copolymer aus Tetrafluorethylen und Hexafluorpropylen ist.

3. Reaktor nach Anspruch 1 oder 2, **dadurch gekennzeichnet, dass** die Dicke der Beschichtung aus fluoriertem Polymer 1 bis 10 mm und vorzugsweise 1,5 bis 5 mm beträgt.

4. Reaktor nach einem der vorhergehenden Ansprüche, **dadurch gekennzeichnet, dass** die Dicke des gelochten Blechs 1 bis 10 mm und vorzugsweise 3 bis 6 mm beträgt.

5. Reaktor nach einem der vorhergehenden Ansprüche, **dadurch gekennzeichnet, dass** die Oberfläche, die mit den Löchern versehen ist, 10 bis 50 % und vorzugsweise 30 bis 40 % der Gesamtoberfläche des gelochten Blechs ausmacht.

6. Reaktor nach einem der vorhergehenden Ansprüche, **dadurch gekennzeichnet, dass** Öffnungen in die metallische Innenwand des Reaktors gebohrt sind.

7. Reaktor nach einem der vorhergehenden Ansprüche, **dadurch gekennzeichnet, dass** das gelochte Blech mit senkrechten Rippen ausgestattet ist.

8. Reaktor nach einem der vorhergehenden Ansprüche, **dadurch gekennzeichnet, dass** auf der Innenwand des Reaktors eine oder mehrere kreisförmige Rillen erzeugt sind.

9. Reaktor nach einem der vorhergehenden Ansprüche, **dadurch gekennzeichnet, dass** die Beschichtung mit Nanoröhrchen aus Kohlenstoff beschickt ist.

10. Verfahren zur Herstellung eines Reaktors nach einem der vorhergehenden Ansprüche, **dadurch gekennzeichnet, dass** es einen Schritt umfasst, in dem die metallische Innenwand des Reaktors mit der unebenen Seite eines gelochten Blechs in Kontakt gebracht wird und die andere freie Seite des Blechs mit der Beschichtung aus fluoriertem Polymer in Kontakt gebracht wird, gefolgt von einem Verankerungsschritt, in dem die Beschichtung aus fluoriertem Polymer in die Löcher des Blechs eindringt und unter der Einwirkung der Hitze und des Drucks auf der Innenwand des Reaktors zum Aufliegen kommt.

11. Verfahren zur Fluorierung in flüssiger Phase, **dadurch gekennzeichnet, dass** ein Reaktor nach einem der Ansprüche 1 bis 9 eingesetzt wird.
